# EUROPÄISCHE PATENTSCHRIFT

(11) **EP 2 920 790 B1**
(45) Veröffentlichungstag und Bekanntmachung des Hinweises auf die Patenterteilung: **24.08.2016**
(21) Anmeldenummer: 12808261.7
(22) Anmeldetag: 21.12.2012
(51) Int. Cl.: G21K 1/02

(54) **RÖNTGENSTRAHLUNGSQUELLE, KOLLIMATOR, RÖNTGENOLOGISCHER ARBEITSPLATZ UND VERFAHREN ZUM BETRIEB DERSELBEN**
X-RAY RADIATION SOURCE, COLLIMATOR, RADIOLOGICAL WORKSTATION AND METHOD FOR OPERATING SAME
SOURCE DE RAYONS X, COLLIMATEUR, POSTE DE TRAVAIL RADIOLOGIQUE ET PROCÉDÉ PERMETTANT LEUR FONCTIONNEMENT

(30) Priorität: 16.11.2012 WO PCT/EP2012/004771
(43) Veröffentlichungstag der Anmeldung: 23.09.2015
(73) Patentinhaber: Böhme Management GmbH, 06188 Landsberg OT Peißen (DE)
(72) Erfinder: BÖHME, Karl-Heinz, 06184 Kabelsketal (DE)
(74) Vertreter: Hecht, Jan-David
(86) Internationale Anmeldenummer: PCT/EP2012/005319
(87) Internationale Veröffentlichungsnummer: WO 2014/075700

(56) Entgegenhaltungen:
- DE-A1- 10 327 293
- DE-A1-102004 063 710
- DE-C1- 19 905 823

## Beschreibung

Die vorliegende Erfindung betrifft eine Röntgenstrahlungsquelle nach dem Oberbegriff von Anspruch 1, einen Kollimator nach dem Oberbegriff von Anspruch 9, einen röntgenologischen Arbeitsplatz nach dem Oberbegriff von Anspruch 13 sowie ein Verfahren zum Betrieb derselben.

Röntgenstrahlung bezeichnet elektromagnetische Wellen mit Photonenenergien zwischen 100 eV und einigen MeV, entsprechend Wellenlängen zwischen 10⁻⁸ m (10 Nanometer) und etwa 10⁻¹²m (1 Pikometer). Röntgenstrahlung im engeren Sinn heißt dabei Strahlung, die durch Beschleunigung von Elektronen und Auftreffen derselben auf ein Ziel künstlich erzeugt wird. Auf andere Weise entstehende elektromagnetische Wellen im Frequenzbereich der Röntgenstrahlung werden meist eher als Gammastrahlen bezeichnet.

Röntgenstrahlung kann durch zwei verschiedene Vorgänge entstehen. Zum einen durch starke Beschleunigung geladener Teilchen (meistens Abbremsung oder Ablenkung von Elektronen) - das ist die Bremsstrahlung, deren Spektrum kontinuierlich ist und zum anderen durch hochenergetische Übergänge in den Elektronenhüllen von Atomen oder Molekülen. Das ist die charakteristische Röntgenstrahlung. Sie weist stets ein Linienspektrum auf.

Beide Effekte werden in der Röntgenröhre ausgenutzt, in der Elektronen zunächst von einer Glühwendel (Kathode) aus beschleunigt werden (dabei setzen sie keine Röntgenstrahlung frei, weil die Beschleunigung nicht groß genug ist) und anschließend auf die Anode treffen, in der sie stark abgebremst werden. Dabei entsteht Röntgenstrahlung (Bremsstrahlung, mit insgesamt rund 1 % der eingestrahlten Energie) und Wärme (rund 99 %). Außerdem werden durch Elektronenstöße Elektronen aus den Schalen der Metallatome herausgeschlagen. Die Löcher in den Schalen werden durch andere Elektronen aufgefüllt, wobei charakteristische Röntgenstrahlung entsteht. Die Anoden bestehen heute meist aus Keramik, wobei die Stellen, auf welche die Elektronen auftreffen, aus Molybdän, Kupfer oder Wolfram gefertigt sind.

Für den sicheren Betrieb einer Röntgenröhre ist eine passende Abschirmung in einem Gehäuse notwendig. Diese Abschirmung bewirkt einen Schutz der Röhre vor äußerer mechanischer Belastung, die elektrische Isolation für die benötigte Hochspannung und eine Abschirmung der Röntgenstrahlen in unerwünschte Richtungen. Dazu wird beispielsweise Blei verwendet. In der gewünschten Strahlungsrichtung befindet sich ein Austrittsfenster (meist aus Glas oder Berylliumfolie).

Um eine Parallelisierung der erzeugten Röntgenstrahlung zu bewirken, werden Kollimatoren eingesetzt. Die einfachste Art des Kollimators ist ein Block aus einem Abschirmmaterial, wie zum Beispiel Blei, mit vielen dünnen, geraden Bohrungen. Nur Röntgenstrahlung, die annähernd in Richtung der Bohrungen verläuft, kann den Kollimator passieren, alle anderen Röntgenstrahlen werden absorbiert. Je nach Ausführung (Dicke des Blocks und Durchmesser der Bohrungen) und nach Art des ursprünglichen Röntgenstrahlenbündels gelangt zum Beispiel weniger als ein Prozent der einfallenden Strahlung durch den Kollimator. Weiterhin ist zumeist am Kollimator eine Strahlformungsblende (Kollimatorblende) vorgesehen, die die Strahlung in ihrer lateralen Erstreckung begrenzt, wobei in Bezug auf den Zentralstrahl zumeist zwei Blendenpaare symmetrisch und spiegelbildlich geöffnet bzw. geschlossen werden.

Eine Röntgenstrahlungsquelle weist somit üblicherweise eine Röntgenröhre, ein Gehäuse, Abschirmmittel, eine Strahlformungsblende und einen Kollimator auf.

Röntgenstrahlungsquellen kommen in vielfältiger Weise zum Einsatz, beispielsweise in röntgenologischen Arbeitsplätzen zur Diagnostik und bei der Therapie in der Human- und der Tiermedizin. Dabei kommt beispielsweise eine von einem Röntgendetektor beabstandbare Röntgenstrahlungsquelle zum Einsatz, um bestimmte Objekte in einem menschlichen oder tierischen Körper zu untersuchen und insbesondere dessen Lage festzustellen. Solche Arbeitsplätze weisen horizontal oder vertikal zueinander ausgerichtete Röntgenstrahlungsquellen und Röntgendetektoren auf. Bekannt sind auch sogenannte C-Bögen, die im Raum verschwenkbar angeordnet sind und die einen festen Abstand zwischen Röntgenstrahlungsquelle und Röntgendetektor besitzen.

Aus der DE-C1-199 05 823 ist eine Röntgenstrahlungsquelle bekannt, bei der zumindest ein asymmetrisch einstellbares Blendenpaar vorhanden ist, dessen Blendenblätter sich in lateraler und axialer Richtung erstrecken. Nachteilig an solchen Röntgenstrahlungsquellen ist die damit einhergehende Strahlungsbelastung, die so gering wie möglich gehalten werden muss, da Röntgenstrahlung ionisierend wirkt und dadurch Veränderungen im lebenden Organismus hervorrufen und Schäden bis hin zu Krebs verursachen kann. Deshalb ist beim Umgang mit der Röntgenstrahlung der Strahlenschutz zu beachten.

Die Aufgabe der vorliegenden Patentanmeldung besteht darin, eine Röntgenstrahlungsquelle bereit zu stellen, die flexibler und/oder mit verringerter Strahlungsbelastung einsetzbar ist.

Diese Aufgabe wird gelöst mit einer Röntgenstrahlungsquelle nach Anspruch 1, einem Kollimator nach Anspruch 7, einen röntgenologischen Arbeitsplatz nach Anspruch 11 und einem Verfahren zum Betrieb derselben nach Anspruch 12. Vorteilhafte Weiterbildungen sind in den abhängigen Unteransprüchen angegeben.

Die Erfinder haben erkannt, dass die erfindungsgemäße Aufgabe überraschend dadurch gelöst werden kann, wenn eine Strahlformungsblende eingesetzt wird, die zumindest ein Blendenpaar aufweist, das in Bezug auf einen Zentralstrahl der Röntgenröhre asymmetrisch einstellbar ist. "Asymmetrisch" heißt in diesem Zusammenhang, dass der Abstand der beiden Blendenelemente des Blendenpaares einen unterschiedlichen Abstand vom durchgehenden Zentralstrahl haben. Dadurch lässt sich die Strahlenbelastung sehr gering in denjenigen Teilen halten, die nicht untersucht werden soll. Beispielsweise kann so mittels einer Durchleuchtung das menschliche Fußgelenk, auf das der Zentralstrahl eingerichtet ist, zugleich mit dem Schienbein untersucht werden ohne dass jenseits des Fußes Röntgenstrahlung auftritt. Die Ausrichtung des Zentralstrahls auf das Fußgelenk ist deshalb erforderlich, weil nur der Zentralstrahl verzerrungsfrei abbildbar ist. Mit bisher bekannten Röntgenstrahlungsquellen war es zur gleichzeitigen Abbildung des Schienbeins erforderlich, die Kollimatorblende aufzublenden, so dass aufgrund der symmetrischen Kollimatorblendenelementverstellung ein dem Abstand Fußgelenk-Schienbein entsprechender Bereich jenseits des Fußgelenks zugleich mitbestrahlt worden wäre. Ein anderes Anwendungsfeld besteht beispielsweise in der Urologie, und zwar in der Untersuchung einer einzelnen Niere, wobei der Zentralstrahl auf diese Niere eingerichtet wird und über das asymmetrisch geöffnete Blendenpaar zugleich noch die angrenzenden Nierengefäße abgebildet werden können.

In einer bevorzugten Ausgestaltung ist vorgesehen, dass zumindest zwei Blendenpaare vorgesehen sind, die in unterschiedlichen Richtungen einstellbar sind. Dann lässt sich der Röntgenstrahl um den Zentralstrahl herum optimal anforderungsgemäß modellieren. Anstelle von beispielsweise zwei senkrecht zueinander angeordneten Blendenelementpaaren können auch drei oder mehrere Blendenelementpaar verwendet werden, die in regelmäßigen Winkelabständen zueinander angeordnet sind, wenn statt rechteckigen Strahlquerschnitten solche mit rundem bzw. elliptischen oder allgemein gebogenen Strahlquerschnitten verwendet werden sollen.

Zweckmäßig sind die Blendenelemente unabhängig von einander separat ansteuer- und einstellbar, wozu bevorzugt verschiedene Motoren verwendet werden.

Zweckmäßig ist die Strahlformungsblende am Kollimator als Kollimatorblende angeordnet, wodurch keine zusätzliche Strahlformungsblende erforderlich ist, und die Röntgenstrahlungsquelle sehr kompakt gehalten werden kann.

In einer vorteilhaften Weiterbildung ist vorgesehen, dass der Kollimator ein Kollimationsmittel aufweist und die Strahlformungsblende zwischen Röntgenröhre and Kollimationsmittel angeordnet ist. Dann erhöht sich die Wirksamkeit der Strahlformung, weil gegebenenfalls die Strahlformungsblende ungewollt passierende Röntgenstrahlung durch die Kollimationsmittel ausgelöscht wird. Als Kollimationsmittel kann beispielsweise ein Block aus einem Abschirmmaterial, wie zum Beispiel Blei, mit vielen dünnen, geraden Bohrungen verwendet werden.

Vorteilhaft ist die Strahlformungsblende, insbesondere die Kollimatorblende im Bereich des Fokus der Röntgenröhre angeordnet, um Streustrahlung zu vermeiden. Dann kann der Kollimator bzw. die Röntgenstrahlungsquelle sehr kompakt gehalten werden.

Bevorzugt ist vorgesehen, dass die Strahlformungsblende gegenüber der Röntgenröhre verdrehbar angeordnet ist, wobei die Verdrehbarkeit insbesondere um die Zentralstrahlachse der Röntgenröhre besteht. Bisher wurde das gesamte Kollimatorgehäuse verdreht, um Blenden auf das zu untersuchende Objekt auszurichten, was aber daher nachteilig ist, weil dann am Gehäuse befindliche Bedienelemente ggf. nicht mehr erreichbar sind. Mit dieser Lösung kann die Blende unabhängig vom Kollimatorgehäuse verstellt werden, so die Bedienelemente stets in derselben Position erreichbar sind.

In diesem Zusammenhang wird zweckmäßiger Weise ein einzelner Motor verwendet, der die Winkelverstellung der Strahlungsblende gegenüber der Röntgenröhre ermöglicht.

Besonders zweckmäßig ist es, wenn sich zumindest ein Blendenelement in lateraler und axialer Richtung erstreckt. Dann kann das Blendenelement über den lateralen Bereich gut mechanisch geführt werden, um die Verstellung zu erleichtern und das laterale Blendenelement kann ggf. auch zur zusätzlichen Abschirmung verwendet werden, wenn es aus einem Abschirmmaterial besteht. Der axiale Blendenelementbereich dient der Ausblendung selbst und ist vorzugsweise konisch ausgebildet, wobei die Konizität entsprechend der Fokuslänge gewählt wird, um Streustrahlen zu vermeiden.

Wenn die sich axial erstreckenden Bestandteile zweier benachbart angeordneter Blendenelemente verschränkbar ausgebildet sind, wird ein unerwünschtes Austreten von Röntgenstrahlung, insbesondere von Streustrahlung verhindert. Insbesondere ist vorgesehen, dass die sich axial erstreckenden Bestandteile der benachbart angeordneten Blendeelemente Zähne aufweisen, die miteinander kämmen, wodurch eine besonders einfache Art der Verschränkung ermöglicht wird.

Besonders bevorzugt ist vorgesehen, dass die Strahlformungsblende eine in Abhängigkeit von der Entfernung zu einer der Röntgenstrahlungsquelle zugeordneten Röntgendetektor veränderliche Öffnung aufweist, wobei die Strahlformungsblende insbesondere so ansteuerbar ist, dass sie sich mit zunehmender Entfernung schließt. Dann kann die Strahlenbelastung für das Objekt minimal gehalten werden. Dabei ist zweckmäßig vorgesehen, dass die Blendenöffnung so eingestellt wird, dass eine optimale Ausleuchtung des Röntgendetektors erfolgt.

Sicherheit und Effizienz der Röntgenstrahlungsquelle kann dadurch erhöht werden, dass eine Visiereinrichtung vorgesehen ist zur Ausrichtung in Bezug auf das zu untersuchende Objekt bzw. einen dieser Röntgenstrahlungsquelle zugeordneten Röntgendetektor, wobei die Visiereinrichtung bevorzugt so ausgebildet ist, dass der Betrieb dieser Röntgenstrahlungsquelle davon abhängig ist, ob diese Röntgenstrahlungsquelle mit diesem Röntgendetektor fluchtet. "Fluchten" meint in diesem Zusammenhang jedes gezielte Einstrahlen von Röntgenstrahlen aus einer Röntgenquelle auf einen Röntgendetektor, d.h. sowohl eine Ausrichtung des Zentralstrahls der Röntgenquelle auf das Zentrum des Röntgendetektors, als auch beispielsweise eine Ausrichtung des Zentralstrahls auf den Rand des Röntgendetektors. Dadurch wird zum einen sichergestellt, dass nur dann ein Betrieb der Röntgenstrahlungsquelle möglich ist, wenn die Röntgenstrahlung auf den Röntgendetektor trifft, wodurch sich die Sicherheit erhöht. Andererseits wird durch die gezielte Ausrichtung des Zentralstrahls in Bezug auf den Röntgendetektor die Effizienz erhöht.

Die Visiereinrichtung weist vorteilhaft eine LASER-Strahlungsquelle auf, wodurch sich eine hohe Genauigkeit ergibt. Alternativ oder zusätzlich kann eine sogenannte Power-LED zum Einsatz kommen, um Gesundheitsschäden infolge der LASER-Bestrahlung zu verhindern, beispielsweise, wenn auf ein menschliches Gesicht eingerichtet werden muss. Wenn beide Strahlungsquellen vorgesehen sind, dann ist es bevorzugt, wenn die jeweilige Strahlungsquelle selektiv ausgewählt werden kann.

Zur Erhöhung des Komforts und auch zur Senkung der Strahlungsbelastung kann eine elektronische Kamera oder Videoaufzeichnungsvorrichtung vorgesehen sein, die das Abbild des zu untersuchenden Objekts aufzeichnet. Dann ist es vorteilhaft, wenn eine Videoausgabevorrichtung vorgesehen ist, die dieses Abbild wiedergibt und auch die Position der einzelnen Blendenelemente der Strahlformungsquelle und/oder die Position des Zentralstrahls, wozu eine entsprechende Verarbeitungs- und Ansteuervorrichtung vorgesehen ist. Die Position der einzelnen Blendenelemente kann beispielsweise direkt an den jeweiligen Motoren ausgelesen werden, wenn es sich um Schrittmotoren handelt.

Dadurch ergeben sich zahlreiche Vorteile. Zum einen kann die Röntgenstrahlungsquelle bzw. der Kollimator wesentlich kompakter aufgebaut werden, weil zusätzliche Leuchtmittel und Spiegel sowie eigene korrespondierend zur Öffnung der Strahlformungsblende schließende Blenden zur optischen Simulation der Strahlformungsblendenöffnung auf dem zu untersuchenden Objekt nicht mehr erforderlich sind.

Zum anderen wird eine exakte Ausrichtung des Zentralstrahls und Einrichtung der Strahlformungsblende auch bei schlechten Lichtverhältnissen und insbesondere in sehr heller Umgebung ermöglicht.

Schließlich kann das Abbild des zu untersuchenden Objektes zusammen mit der überlagerten Strahlformungsblendeneinstellung ggf. auch extern, beispielsweise an entfernt stehende Bildschirme ausgegeben werden.

Dadurch wird zum einen die Strahlungsbelastung reduziert, denn nun muss für eine Justierung von Röntgenstrahlungsquelle und -detektor in Bezug auf das Objekt kein Dauerbetrieb bzw. Betrieb mit normaler Leistung gefahren werden, sondern es ist möglich, in einem ersten Schritt eine Orientierungsaufnahme mit verringerter Dosis vorzunehmen, in einem zweiten Schritt Zentralstrahl und Blenden auf dem Bildschirm also auf dem Objekt direkt nachzujustieren und in einem dritten Schritt die gewünschte Aufnahme mit normaler Dosis zu fahren.

Andererseits können so die Röntgenstrahlungsquelle und ein daraus bestehender röntgenologischer Arbeitsplatz ihrerseits extern, beispielsweise aus einem benachbarten Raum angesteuert werden. Eine solche externe Ansteuerung erfolgt über eine geeignete Datenleitung, wie eine CAN-Busleitung.

Zweckmäßig ist unterhalb der Strahlformungsblende, also bevorzugt unter der Kollimatorblende zumindest ein in den Röntgenstrahlengang einbringbarer Filter angeordnet, wobei der Filter insbesondere Teil eines Filtermagazins ist, in dem mehrere verschiedenen Filter angeordnet sind. So kann ein Rundfiltermagazin mit drei Filterfenstern und einem vierten Fernster ohne Filter verwendet werden oder auch ein Schieber mit solchen vier Fenstern. Bei den Filtern kann es sich beispielsweise um 0,2 mm Kupfer handeln, das für die Bestrahlung von Kindern eingesetzt wird, um die Strahlung härter zu machen. Andere Filter sind beispielsweise Filter aus Aluminium und aus Aluminium und Kupfer kombiniert. Mit den Filtern das Röntgenstrahlspektrum gezielt beeinflusst.

An der Strahlenaustrittsseite der Röntgenstrahlungsquelle, also insbesondere unterhalb des Austrittsfensters des Kollimators, sind bevorzugt Mittel zur Anordnung von zusätzlichen Filtern, wie zum Beispiel Verlaufsfilter und dgl. angeordnet.

Vorzugsweise weist die Röntgenstrahlungsquelle ein Messgerät für das Dosisflächenprodukt auf, das insbesondere im Kollimator vor dem Austrittsfenster angeordnet ist

Insgesamt wird auch unabhängiger Schutz beansprucht für einen röntgenologischen Arbeitsplatz, umfassend zumindest eine erfindungsgemäße Röntgenstrahlungsquelle, einen Röntgenstrahlungsdetektor, eine Vorrichtung zur Definition einer Anordnungsposition für ein zu untersuchendes Objekt, beispielsweise eine Patientenliege oder dgl., eine geeignete Verarbeitungs- und Ansteuervorrichtung sowie eine Ausgabevorrichtung, beispielsweise einen Drucker, Speichermittel oder einen Bildschirm.

Hinsichtlich der Vorrichtung zur Definition einer Anordnungsposition für ein zu untersuchendes Objekt wird auf den Offenbarungsgehalt der am 21.12.2012 von demselben Anmelder eingereichten PCT-Anmeldung (T 59761 WO) "Röntgentisch und röntgenologischer Arbeitsplatz damit" verwiesen, deren Inhalt somit vollumfänglich einbezogen wird.

Weiterhin auch unabhängiger Schutz beansprucht den vorstehend definierten röntgenologischen Arbeitsplatz, bei dem allerdings nicht zwingend die erfindungsgemäße Röntgenstrahlungsquelle eingesetzt wird. Auf jeden Fall soll eine elektronische Kamera oder Videoaufzeichnungsvorrichtung zur Aufnahme einer Abbildung des gewünschten Bestrahlungsbereichs auf dem Objekt vorgesehen sein und eine Ausgabeeinheit, beispielsweise in Form eines Bildschirms, in der die optische Aufnahme mit a) der simulierten Lage und Ausrichtung der Strahlformungsblende und Lage des Zentralstrahls und/oder mit b) der röntgenologischen Aufnahme des Objekts überlagert werden kann. Dann kann im Fall a) die Ausrichtung von Zentralstrahl und die Blendenlage besonders schnell auf den gewünschten Objektbereich erfolgen. In Fall b) ist es möglich, die vorstehend genannten drei Schritte der Justierung und Aufnahme vornehmen, so dass noch einmal ein exakter Abgleich des zu bestrahlenden Objektbereichs mit der tatsächlichen Lage eines in dem Objekt zu untersuchenden Elements vorgenommen werden kann. Die Simulation, Übertragung und Überlagerung der entsprechenden Informationen erfolgt mittels einer geeigneten Berechnungs-, Auswerte-, Übertragungs-und Ansteuereinheit.

Für diese erfindungsgemäße Ausgestaltung ist die asymmetrische Blendenverstellmöglichkeit nicht zwingend erforderlich, allerdings erhöht sie natürlich die Flexibilität und verringert die Strahlenbelastung der Bestrahlung.

Unabhängiger Schutz wird auch beansprucht für den erfindungsgemäßen Kollimator für eine Röntgenstrahlungsquelle, der sich dadurch auszeichnet, dass der Kollimator zumindest eine Kollimatorblende aufweist, die zumindest ein Blendenpaar aufweist, das in Bezug auf einen Zentralstrahl der Röntgenröhre asymmetrisch einstellbar ist. Die Kollimatorblende besteht insbesondere aus einem für die Röntgenstrahlung abschirmenden Material, beispielsweise Blei.

Zusätzlich kann der Kollimator die auf den Kollimator bezogenen Merkmale der erfindungsgemäßen Röntgenstrahlungsquelle aufweisen.

Insbesondere ist es vorteilhaft, wenn am Kollimator eine elektronische Kamera oder Videoaufzeichnungsvorrichtung zur Aufzeichnung einer Abbildung angeordnet ist und auch eine Bildausgabeeinheit in Form eines Bildschirms, die bevorzugt als Touchpanel ausgebildet ist. Dann kann die Ausrichtung und Nachjustierung in Bezug auf das zu untersuchende Objekt direkt an der Röntgenstrahlungsquelle erfolgen.

Weiterhin wird unabhängiger Schutz beansprucht für das erfindungsgemäße Verfahren zum Betrieb einer Röntgenstrahlungsquelle und/oder eines Kollimators einer Röntgenstrahlungsquelle, das sich dadurch auszeichnet, dass zumindest zwei Blendenelemente einer Strahlformungsblende in Bezug auf einen Zentralstrahl einer Röntgenröhre asymmetrisch eingestellt werden. Als Röntgenstrahlungsquelle bzw. Kollimator kommen bevorzugt die erfindungsgemäße Röntgenstrahlungsquelle und der erfindungsgemäße Kollimator zum Einsatz.

Unabhängiger Schutz wird auch für das erfindungsgemäße Verfahren zur Positionierung und/oder Nachjustierung der Röntgenstrahlungsquelle beansprucht.

Die Merkmale der vorliegenden Erfindung und weitere Vorteile werden im Rahmen der Beschreibung bevorzugter Ausführungsbeispiele im Zusammenhang mit den Figuren deutlich werden. Dabei zeigen rein schematisch:
- Fig. 1: den erfindungsgemäßen röntgenologischen Arbeitsplatz in einer Draufsicht von oben,
- Fig. 2: den erfindungsgemäßen röntgenologischen Arbeitsplatz nach Fig. 1 in einer Seitenansicht,
- Fig. 3: den erfindungsgemäßen röntgenologischen Arbeitsplatz nach Fig. 1 und Fig. 2 in einer ersten Verwendung,
- Fig. 4: den erfindungsgemäßen röntgenologischen Arbeitsplatz nach Fig. 1 und Fig. 2 in einer zweiten Verwendung
- Fig. 5: den erfindungsgemäßen röntgenologischen Arbeitsplatz nach Fig. 1 und Fig. 2 in einer dritten Verwendung,
- Fig. 6: den erfindungsgemäßen Kollimator in einer perspektivischen Durchsicht,
- Fig. 7: den erfindungsgemäßen Kollimator nach Fig. 6 in einer Durchsicht von oben und
- Fig. 8: eine Ausschnittsansicht nach Fig. 6 in Durchsicht vom Bereich der Kollimatorblende.

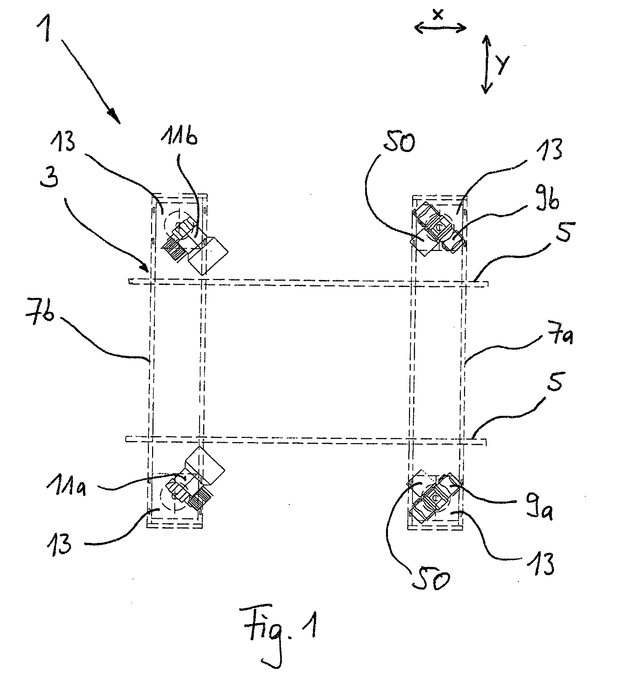

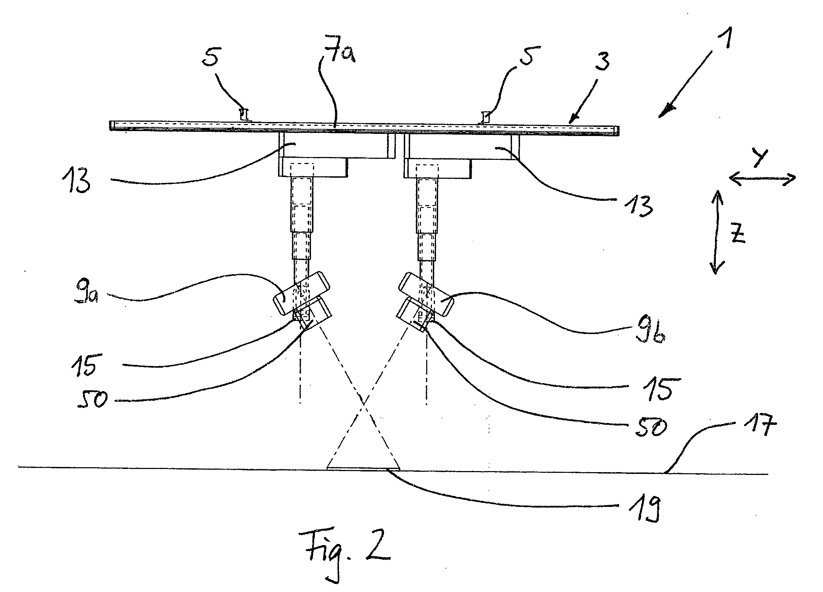

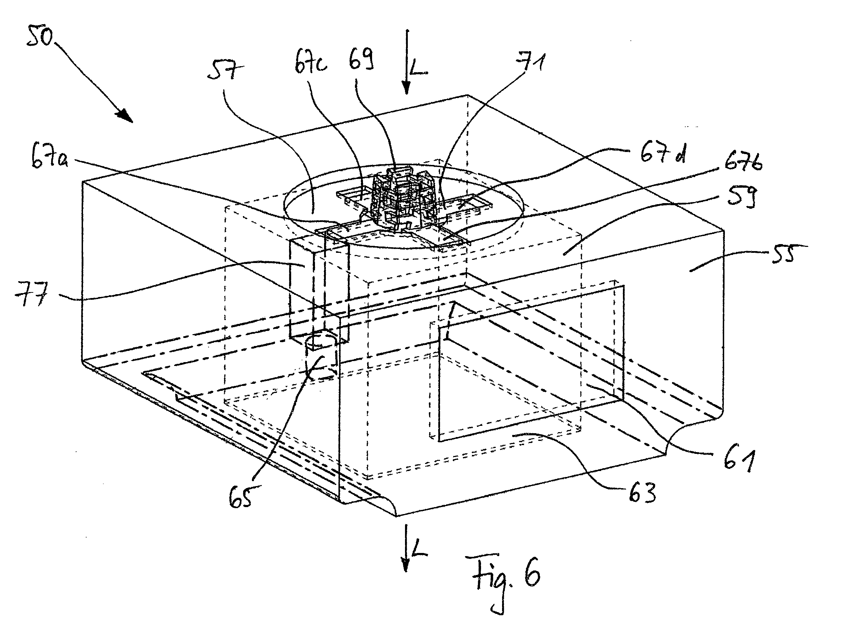

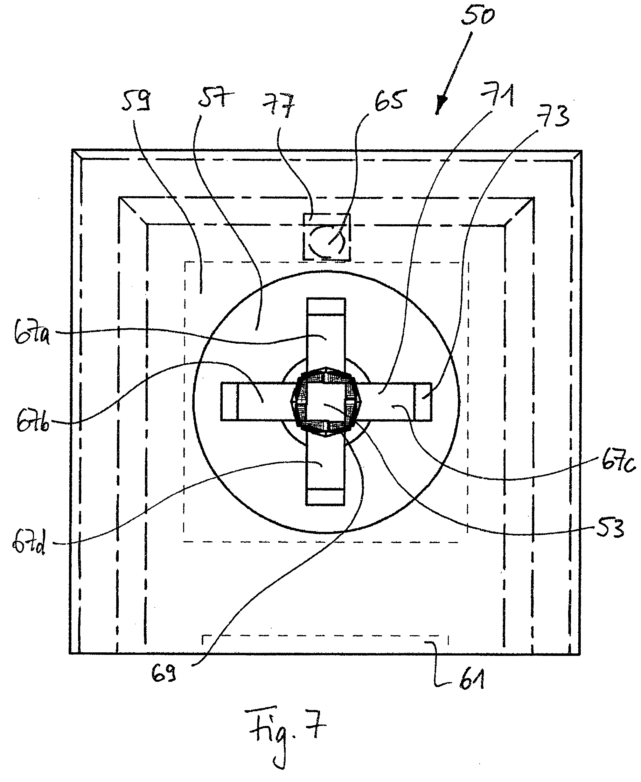

In Fig. 1 und Fig. 2 ist der erfindungsgemäße röntgenologische Arbeitsplatz 1 in einer Draufsicht von oben und in einer Seitenansicht rein schematisch gezeigt, wobei Fig. 6, 7 und 8 den erfindungsgemäßen Kollimator 50 rein schematisch in Durchsichtszeichnungen zeigen.

Es ist zu erkennen, dass der röntgenologische Arbeitsplatz 1 ein strichliert gezeichnetes Schienensystem 3 mit zwei in einer ersten Richtung x verlaufenden Schienen 5 und zwei an diesen Schienen 5 in Richtung x verfahrbar angeordneten Trägerschienen 7a, 7b aufweist, wobei an der einen Trägerschiene 7a zwei Röntgenstrahlungsquellen 9a, 9b und an der anderen Trägerschiene 7b zwei Röntgenstrahlungsdetektoren 11a, 11b, wobei die Röntgenstrahlungsquellen 9a, 9b und auch die Röntgenstrahlungsdetektoren 11a, 11b an den Trägerschienen 7a, 7b jeweils in einer zur Richtung x senkrechten Richtung y verfahrbar angeordnet sind. Das Schienensystem 3 ist an einer Decke befestigt und die Richtungen x, y sind dabei in einer horizontalen Ebene ausgerichtet.

Die Länge der Schienen 5 beträgt beispielsweise ca. 5 m und die Trägerschienen 7a, 7b weisen eine Länge von ca. 4 m und eine Breite von ca. 55 cm auf, wobei der Abstand der Schienen 5 voneinander ca. 1,9 m beträgt. Diese Elemente sind vorteilhaft aus Edelstahl oder Aluminium gefertigt.

Die Anordnung der Röntgenstrahlungsquellen 9a, 9b und der Röntgenstrahlungsdetektoren 11a, 11b an den jeweiligen Trägerschienen 7a, 7b erfolgt dabei, wie in Fig. 2 besonders gut zu erkennen ist, über jeweils einen in einer senkrecht zu den Richtungen x, y orientierten Richtung z teleskopierbaren Vertikalträger 13, an dem die jeweilige Röntgenstrahlungsquelle 9a, 9b bzw. Röntgenstrahlungsdetektor 11a, 11b um eine vertikale Achse 360° verdrehbar und in einer vertikalen Ebene um 360° verschwenkbar angeordnet ist. Hierzu sind jeweilige Verdreh-Verkippungsmittel 15 vorgesehen.

Die Röntgenstrahlungsquellen 9a, 9b sind Röntgenstrahlungsquellen bekannter Bauart mit einer Röntgenröhre (nicht gezeigt), wie sie beispielsweise aus C-Bögen bekannt sind. Die Röntgenstrahlungsdetektoren 11a, 11b beinhalten jeweils einen Röntgenbildverstärker (nicht gezeigt) bekannten Aufbaus und eine Hochgeschwindigkeitsvideokamera (nicht gezeigt), die in der Lage ist, bis zu 250 Bilder/s aufzunehmen.

Wie in Fig. 2 zu erkennen ist, ist am Boden 17 an weiterer Röntgenstrahlungsdetektor 19 angeordnet, dessen Detektorfläche (nicht gezeigt) sich horizontal erstreckt. Dieser Röntgenstrahlungsdetektor 19 kann als Röntgenfolie ausgebildet sein, ist jedoch bevorzugt ebenfalls ein elektronischer Detektor.

Die Röntgenstrahlungsquellen 9a, 9b weisen jeweils einen Kollimator 50 auf, dessen Kollimatorblende 57 aus Blei in ihrer Öffnung 53 exakt und vollautomatisch so an die exakte Entfernung von dem jeweiligen Röntgenstrahlungsdetektor 11a, 11b, 19 anpassbar ist, dass für eine fluchtende Anordnung das ausgestrahlte Strahlenbündel der Röntgenstrahlungsquelle 9a, 9b den Bildbereich des Röntgenstrahlungsdetektors 11a, 11b, 19 abdeckt. Für eine stereotaktische Aufnahme ist vorgesehen, dass der Zentralstrahl einer Röntgenstrahlungsquelle 9a, 9b auf den gegenüberliegenden Bildrand des jeweiligen Röntgenstrahlungsdetektors 11a, 11b, 19 eingestellt wird und der Kollimator 50 so eingestellt ist, dass das ausgestrahlte Strahlenbündel bis zum anderen Bildrand reicht. Zur Verringerung der Strahlenbelastung ist zusätzlich vorgesehen, dass die Kollimatorblende 57 in zumindest einer Richtung so asymmetrisch verfahrbar ist, dass der neben dem Zentralstrahl befindlich Teil des Strahlenbündels, der nicht auf den Röntgenstrahlungsdetektor 11a, 11b, 19 fallen würde, ausgeblendet wird. Vorteilhaft ist die Kollimatorblende 51 in beiden Richtungen asymmetrisch einstellbar. Für die vollautomatische Betätigung der Kollimatorblende 51 wird die genaue Lage der Elemente zueinander im Raum mittels besonderer Positionssensoren (nicht gezeigt) bestimmt.

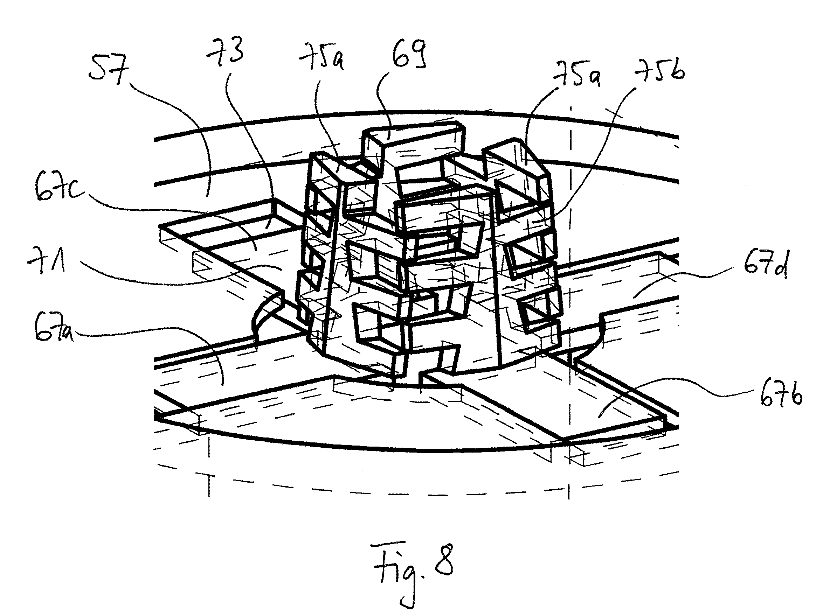

Genauer gesagt weist der Kollimator 50 ein Kollimatorgehäuse 55, eine Kollimatorblende 57, ein Kollimationsmittel 59 in Form eines Bleiblocks mit Durchgangsbohrungen in axialer Richtung L (Längsachse), eine Displayeinheit 61, die berührungsempfindlich ausgebildet ist und der Eingabe von Parametern, wie der Form der Öffnung 53 dient, ein Austrittsfenster 63 und eine Visiervorrichtung 65 auf. Die Kollimatorblende weist vier Blendenelemente 67a, 67b, 67c, 67d auf, wovon jeweils zwei 67a, 67b, 67c, 67d gegenüberliegend angeordnet sind. Die Blendenelemente 67a, 67b, 67c, 67d weisen in Bezug auf die Längsachse L jeweils axiale Blendenelementbereiche 69 und laterale Blendenelementbereiche 71 auf. Die lateralen Blendenelementbereiche 71 werden in entsprechenden Führungen 73 geführt und sind darin mithilfe eines jeweils zugeordneten Motors (nicht gezeigt) gezielt lateral, also senkrecht zur Längsachse L verfahrbar. Die axialen Blendenelementbereiche 69 von benachbart angeordneten Blendenelementen 67a, 67b, 67c, 67d sind verschränkt zueinander angeordnet und kämmen mit beidseits angeordneten Zähnen 75a, 75b (vgl. Fig. 8). Dadurch ist über den gesamten Verfahrbereich aller Blendenelement 67a, 67b, 67c, 67d eine Überlappung der axialen Blendenelementbereiche 69 der jeweilig benachbarten Blendenelemente 67a, 67b, 67c, 67d sichergestellt, so dass die Röntgenstrahlung wirksam ausgeblendet wird. Was noch dadurch unterstützt wird, dass die axialen Blendenelementbereiche 69 konisch sich in Strahlrichtung erweiternd ausgebildet sind, um Streustrahlung zu vermeiden, und die Kollimatorblende 57 sehr nah am Fokus der Röntgenröhre angeordnet ist, wobei die Konizität entsprechend ausgewählt ist. Die lateralen Blendenelementbereiche 71 bewirken eine zusätzliche Abschirmung der Röntgenstrahlung und stellen außerdem die sichere laterale Führung der Blendenelemente 67a, 67b, 67c, 67d sicher.

Durch diese Ausgestaltung der Kollimatorblende 57 wird bewirkt, dass der Röntgenstrahl, der durch das Austrittsfenster 63 tritt, einen der Öffnung 53 entsprechenden Querschnitt aufweist, wobei der Röntgenstrahl aufgrund des Durchtritts durch das Kollimationsmittel 59 hochgradig parallelisiert ist. Zusätzlich ist die Kollimatorblende 57 gegenüber dem Kollimatorgehäuse 55 drehbar angeordnet, und zwar in einer Ebene senkrecht zur Längsachse L. Zur Einstellung der Winkelausrichtung der Kollimatorblende 57 gegenüber dem Kollimatorgehäuse 55 ist ein weiterer motorischer Antrieb (77) vorgesehen. Da das Kollimatorgehäuse jeweils fest mit den Röntgenstrahlungsquellen 9a, 9b verbunden ist, erfolgt über das Verdrehen der Kollimatorblende 57 also eine Verdrehung der Kollimatorblende 57 gegenüber der Röntgenröhre, so dass die Ausrichtung der durch die Öffnung 53 definierten Strahlform auf dem zu untersuchenden Objekt variiert werden kann, ohne das Kollimatorgehäuse 55 bzw. die jeweilige Röntgenstrahlungsquelle 9a, 9b zu verdrehen, wodurch die Displayeinheit 61 für den Bediener (nicht gezeigt) immer optimal erreichbar ist.

Weiterhin sind folgende, nicht näher gezeigte Elemente an dem erfindungsgemäßen röntgenologischen Arbeitsplatz 1 vorhanden: ein Laufband sowie eine Befestigungsvorrichtung, die beide am Boden 17 angeordnet sind. Außerdem sind Anzeigegeräte zur Auswertung vorhanden und es ist eine Steuer- und Auswertevorrichtung vorgesehen, um die vorzunehmenden Messungen durchzuführen und anschließend oder währenddessen eine Analyse vornehmen zu können. An jeder Röntgenstrahlungsquelle 9a, 9b ist ein optisches Visiermittel 65 in Form eines Laserstrahlers angeordnet und an den Röntgenstrahlungsdetektoren 11a, 11b jeweils ein oder mehrere Lichtdetektoren (nicht gezeigt), um festzustellen, ob eine Röntgenstrahlungsquelle 9a, 9b auf den gewünschten Röntgenstrahlungsdetektor 11, 19 optimal ausgerichtet ist. Dabei kann es sich um eine fluchtende Ausrichtung handeln, wie sie für dreidimensionale Aufnahmen erforderlich ist, oder um eine auf ein Objekt fokussierte Ausrichtung, wie sie für stereotaktische Aufnahmen erforderlich ist.

Außerdem sind elektronische Bildaufzeichnungsmittel (nicht gezeigt) an den Röntgenstrahlungsquelle 9a, 9b angeordnet, vorzugsweise zur Bewegtbildaufnahme eines zu untersuchenden Objektes. Im Zusammenhang mit einer Ansteuer- und Verarbeitungsvorrichtung (nicht gezeigt) können dann auf der Displayeinheit 61 sowohl das statische bzw. Bewegtbild des zu untersuchenden Objektes als auch die genaue Position des Zentralstrahls der jeweiligen Röntgenstrahlungsquelle 9a, 9b und die genaue Form der Öffnung 53, also die Lage der Blendenelemente 67a, 67b, 67c, 67d angezeigt werden. Weiterhin kann auf der Displayeinheit 61 auch die Röntgenbildaufnahme wiedergegeben werden. Schließlich lassen sich auf der Displayeinheit 61 auch diese Informationen überlagert ausgeben.

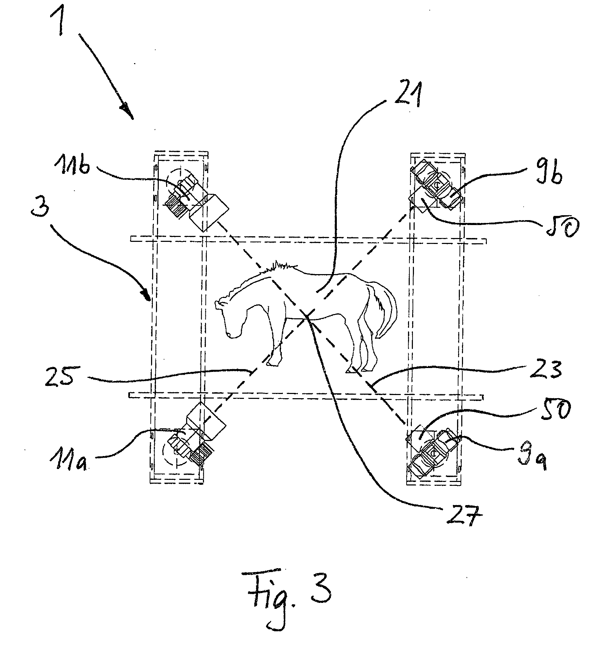

In Fig. 3 ist ein erster bevorzugter Anwendungsfall näher gezeigt. Dabei steht ein Pferd 21 in dem erfindungsgemäßen röntgenologischen Arbeitsplatz 1 auf dem Laufband und ist so befestigt, dass es sich zwar bewegen, beispielsweise traben, jedoch das Laufband nicht verlassen kann.

Die linke Röntgenstrahlungsquelle 9a ist mit Hilfe der Visiervorrichtung fluchtend auf den rechten Röntgenstrahlungsdetektor 11b ausgerichtet und die rechte Röntgenstrahlungsquelle 9a ist mit Hilfe der Visiervorrichtung fluchtend auf den linken Röntgenstrahlungsdetektor 11b ausgerichtet (d.h. die Zentralstrahlen der beiden Röntgenstrahlungsquellen 9a, 9b sind auf das Zentrum der jeweiligen Röntgenstrahlungsdetektoren 11a, 11b ausgerichtet). Die Visiervorrichtung ist dabei so mit der Steuer- und Auswerteeinheit verbunden, dass ein Betrieb der Röntgenstrahlungsquellen 9a, 9b nur dann möglich ist, wenn die Fluchtung vorliegt, wobei in diesem Fall die beiden Fluchtungsstrahlen 23, 25 jeweils zentral auf die beiden Röntgenstrahlungsdetektoren 11b, 11a eintreffen. Die beiden Fluchtungsstrahlen 23, 25 kreuzen sich in einem Schnittpunkt 27, in dem das Pferd 21 auf dem Laufband steht, d.h. die beiden Fluchtungsstrahlen 23, 25 wurden in den Richtungen x, y, z so ausgereichtet, dass der Schnittpunkt 27 auf das interessierende Teil des Pferdes, beispielsweise ein Gelenk ausgerichtet ist. (Die genaue Ausrichtung des Schnittpunktes 27 auf das Pferd 21 ist hier nicht exakt gezeigt.)

Das Laufband ist mit Bodenkraftsensoren ausgerüstet, um ein bestimmtes Bewegungsmuster des Pferdes zu erkennen und auch einen Startpunkt und eine Endpunkt dieses Bewegungsmusters zu erkennen. Bei dem Bewegungsmuster kann es sich beispielsweise um ein Traben oder dgl. handeln. Alternativ oder zusätzlich können für dieses Erkennen Beschleunigungssensoren und/oder eine Videokammara verwendet werden.

Sobald das Bewegungsmuster und der Startpunkt erkannt wurden, wird die röntgenologische Durchleuchtungsaufnahme gestartet und kontinuierlich durchgeführt und nach Erkennen des Endpunktes wird die Durchleuchtung beendet. Üblicherweise wird die Durchleuchtung für zumindest zwei, bevorzugt zumindest drei und insbesondere fünf Wiederholungen des Bewegungsmusters durchgeführt. Dadurch wird eine definierte Durchleuchtung sichergestellt und gleichzeitig die Strahlenbelastung zu minimieren.

Durch die Analyse der Bewegung können beispielsweise Fehlstände von Gelenken erfasst werden. Bei Menschen können so Knie-, Hüft- und Fußgelenke untersucht werden. Bei Rindern und Schafen kann beispielsweise der Klauenschnitt so optimiert werden, dass die Bewegung optimal verläuft.

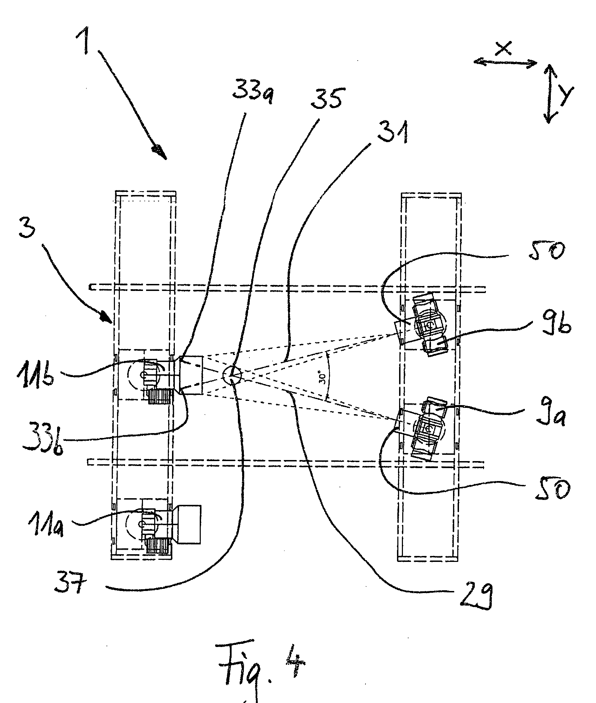

In Fig. 4 ist ein zweiter bevorzugter Anwendungsfall näher gezeigt. Dabei sind die beiden Röntgenstrahlungsquellen 9a, 9b mit ihren Zentralstrahlen 29, 31 auf die jeweils gegenüberliegenden Bildfeldgrenzen 33a, 33b des Röntgenstrahlungsdetektors 11b ausgerichtet, der lotrecht in Bezug auf die Verbindungslinie der beiden Röntgenstrahlungsquellen 9a, 9b angeordnet wird, wobei sich ein gleichseitiges Dreieck zwischen den drei Elementen Röntgenstrahlungsquellen 9a, 9b und Röntgenstrahlungsdetektor 11b aufspannt und die Abstände der Röntgenstrahlungsquellen 9a, 9b vom Röntgenstrahlungsdetektor 11b identisch sind. In den Schnittpunkt 35 der Zentralstrahlen 29, 31 wird das zu untersuchende Objekt 37 verbracht, so dass beide Röntgenstrahlungsquellen 9a, 9b auf dieses Objekt 37 fokussiert sind. Die Zentralstrahlen der beiden Röntgenstrahlungsquellen 9a, 9b scheiden sich unter einem Winkel von 30°, d.h. sie sind in Bezug auf die Normale des Röntgenstrahlungsdetektors 11b mit einem Winkel von jeweils 15° angeordnet. Zur Befestigung des Objektes 37 ist eine reproduzierende Fixierung vorgesehen, so dass stereotaktische Durchleuchtung hoher Qualität vorgenommen werden können. In diesem Fall finden die Durchleuchtungen in einer horizontal verlaufenden Ebene statt. Diese Variante eignet sich vor allem für stehende Objekte.

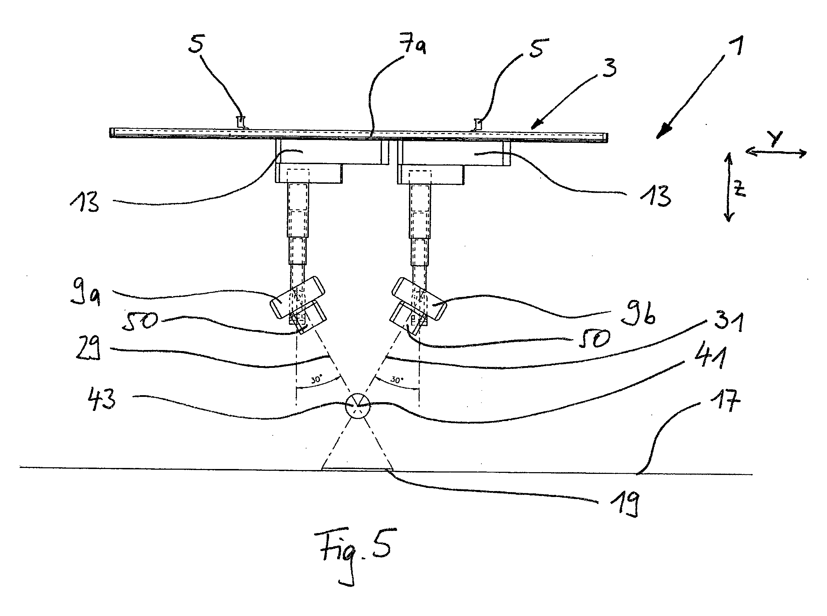

In Fig. 5 ist ein dritter bevorzugter Anwendungsfall näher gezeigt. Dabei sind die beiden Röntgenstrahlungsquellen 9a, 9b mit ihren Zentralstrahlen 29, 31 auf die jeweils gegenüberliegenden Bildfeldgrenzen 39a, 39b des Röntgenstrahlungsdetektors 19 ausgerichtet, wobei die beiden Röntgenstrahlungsquellen 9a, 9b so verschwenkt sind, dass sie zusammen mit dem Röntgenstrahlungsdetektor 19 eine vertikal ausgerichtete Ebene aufspannen und sich ein gleichseitiges Dreieck zwischen den drei Elementen Röntgenstrahlungsquellen 9a, 9b und Röntgenstrahlungsdetektor 19 aufspannt und die Abstände der Röntgenstrahlungsquellen 9a, 9b vom Röntgenstrahlungsdetektor 19 identisch sind. In den Schnittpunkt 41 der Zentralstrahlen 29, 31 wird durch genaue Justierung in den Richtungen x, y, z das zu untersuchende Objekt 43 verbracht, so dass beide Röntgenstrahlungsquellen 9a, 9b auf dieses Objekt 43 fokussiert sind. Die Zentralstrahlen der beiden Röntgenstrahlungsquellen 9a, 9b sind in Bezug auf die Normale des Röntgenstrahlungsdetektors 19 mit einem Winkel von 30° angeordnet. Zur Befestigung des Objektes 43 ist wiederum eine reproduzierende Fixierung vorgesehen, so dass stereotaktische Durchleuchtungen hoher Qualität vorgenommen werden können. Diese Variante eignet sich vor allem für liegende Objekte, wobei ggf. eine geeignete Hubeinrichtung, die für die Röntgenstrahlung transparent ausgebildet ist, vorgesehen werden muss, um das Objekt in den Schnittpunkt 41 verbringen zu können.

Aus der vorstehenden Darstellung ist deutlich geworden, dass die erfindungsgemäße Röntgenstrahlungsquelle 9a, 9b mit dem erfindungsgemäßen Kollimator 50 wesentlich flexibler und sicherer einsetzbar ist. Außerdem ist der erfindungsgemäße röntgenologische Arbeitsplatz 1 sehr flexibel einsetzbar und ist im Stande, Durchleuchtungen hoher Qualität zu liefern.

## Patentansprüche

1. Röntgenstrahlungsquelle (9a, 9b), umfassend eine Röntgenröhre, ein Gehäuse, eine Abschirmung, eine Strahlformungsblende (57) und einem Kollimator (50), wobei die Strahl-formungsblende (57) zumindest ein Blendenpaar (67a, 67b, 67c, 67d) aufweist, das in Bezug auf einen Zentralstrahl (29, 31) der Röntgenröhre asymmetrisch einstellbar ist, **dadurch gekennzeichnet, dass** zumindest ein Blendenelement (67a, 67b, 67c, 67d) sich in lateraler (71) und axialer (69) Richtung erstreckt, **dadurch gekennzeichnet, dass** die sich axial erstreckenden Bestandteile (69) zweier benachbart angeordneter Blendenelemente (67a, 67b, 67c, 67d) verschränkbar ausgebildet sind.

2. Röntgenstrahlungsquelle (9a, 9b) nach Anspruch 1, **dadurch gekennzeichnet, dass** zumindest zwei Blendenpaare (67a, 67b, 67c, 67d) vorgesehen sind, die in unterschiedlichen Richtungen einstellbar sind.

3. Röntgenstrahlungsquelle (9a, 9b) nach Anspruch 1 oder 2, **dadurch gekennzeichnet, dass** die Strahlformungsblende (57) am Kollimator (50) als Kollimatorblende angeordnet ist.

4. Röntgenstrahlungsquelle (9a, 9b) nach einem der vorherigen Ansprüche, **dadurch gekennzeichnet, dass** der Kollimator (50) ein Kollimationsmittel (59) aufweist und die Strahlformungsblende (57) zwischen Röntgenröhre and Kollimationsmittel (59) angeordnet ist.

5. Röntgenstrahlungsquelle (9a, 9b) nach einem der vorherigen Ansprüche, **dadurch gekennzeichnet, dass** die Strahlformungsblende (57) gegenüber der Röntgenröhre verdrehbar angeordnet ist, wobei die Verdrehbarkeit insbesondere um die Zentrahlstrahlachse (29, 31) der Röntgenröhre besteht.

6. Röntgenstrahlungsquelle (9a, 9b) nach Anspruch 5, **dadurch gekennzeichnet, dass** die sich axial erstreckenden Bestandteile (69) der benachbart angeordneten Blendeelemente (67a, 67b, 67c, 67d) Zähne (75a, 75b) aufweisen, die miteinander kämmen.

7. Kollimator (50) für die Röntgenstrahlungsquelle (9a, 9b) nach einem der vorherigen Ansprüche, wobei der Kollimator (50) zumindest eine Kollimatorblende (57) aufweist, die zumindest ein Blendenpaar (67a, 67b, 67c, 67d) aufweist, das in Bezug auf einen Zentralstrahl (29, 31) der Röntgenröhre asymmetrisch einstellbar ist, **dadurch gekennzeichnet, dass** zumindest ein Blendenelement (67a, 67b, 67c, 67d) sich in lateraler (71) und axialer (69) Richtung erstreckt, **dadurch gekennzeichnet, dass** die sich axial erstreckenden Bestandteile (69) zweier benachbart angeordneter Blendenelemente (67a, 67b, 67c, 67d) verschränkbar ausgebildet sind.

8. Kollimator (50) nach Anspruch 7, **dadurch gekennzeichnet, dass** zumindest zwei Blendenpaare (67a, 67b, 67c, 67d) vorgesehen sind, die in unterschiedlichen Richtungen einstellbar sind.

9. Kollimator (50) nach einem der Ansprüche 7 oder 8, **dadurch gekennzeichnet, dass** der Kollimator (50) ein Kollimationsmittel (59) aufweist und die Kollimatorblende (57) in Bezug auf die Röntgeneintrittsrichtung vor dem Kollimationsmittel (59) angeordnet ist.

10. Kollimator (50) nach einem der Ansprüche 7 bis 9, **dadurch gekennzeichnet, dass** die Kollimatorblende (57) gegenüber der Röntgenröhre, bevorzugt auch gegenüber dem Kollinationsmittel (59) verdrehbar angeordnet ist.

11. Röntgenologischer Arbeitsplatz (1), umfassend zumindest eine Röntgenstrahlungsquelle (9a, 9b), einen Röntgenstrahlungsdetektor (11a, 11b, 19), eine Vorrichtung zur Definition einer Anordnungsposition für ein zu untersuchendes Objekt (21, 37, 43), beispielsweise eine Patientenliege oder dgl., eine geeignete Verarbeitungs- und Ansteuervorrichtung sowie eine Ausgabevorrichtung (61), beispielsweise einen Drucker, Speichermittel oder einen Bildschirm, **dadurch gekennzeichnet, dass** die Röntgenstrahlungsquelle (9a, 9b) mit den Merkmalen nach einem der Ansprüche 1 bis 6 und/oder der Kollimator (50) mit den Merkmalen nach einem der Ansprüche 7 bis 10 ausgebildet ist.

12. Verfahren zum Betrieb einer Röntgenstrahlungsquelle (9a, 9b), eines Kollimators (50) einer Röntgenstrahlungsquelle (9a, 9b) oder eines röntgenologischen Arbeitsplatzes (1), der zumindest eine Röntgenstrahlungsquelle (9a, 9b), einen Röntgenstrahlungsdetektor (11a, 11b, 19), eine Vorrichtung zur Definition einer Anordnungsposition für ein zu untersuchendes Objekt (21, 37, 43), eine geeignete Verarbeitungs- und Ansteuervorrichtung sowie eine Ausgabevorrichtung (61) aufweist, wobei zumindest zwei Blendenelemente (67a, 67b, 67c, 67d) einer Strahlformungsblende (57) der Röntgenstrahlungsquelle (9a, 9b) in Bezug auf einen Zentralstrahl (29, 31) einer Röntgenröhre asymmetrisch eingestellt werden, **dadurch gekennzeichnet, dass** zumindest ein Blendenelement (67a, 67b, 67c, 67d) sich in lateraler (71) und axialer (69) Richtung erstreckt, wobei sich die axial erstreckenden Bestandteile (69) zweier benachbart angeordneter Blendenelemente (67a, 67b, 67c, 67d) verschränken.

13. Verfahren nach Anspruch 12, **dadurch gekennzeichnet, dass** die Röntgenstrahlungsquelle (9a, 9b) mit den Merkmalen nach einem der Ansprüche 1 bis 8 und/oder der Kollimator (50) mit den Merkmalen nach einem der Ansprüche 9 bis 12 ausgebildet ist.

## Claims

1. An X-ray emitter (9a, 9b), comprising an X-ray tube, a housing, a shield, a beam forming aperture (57) and a collimator (50), wherein the beam forming aperture (57) includes at least one aperture pair (67a, 67b, 67c, 67d) which is asymmetrically adjustable with reference to a central beam (29, 31) of the X-ray tube, **characterized in that** at least one aperture element (67a, 67b, 67c, 67d) extends in a lateral direction (71) and in axial direction (69), **characterized in that** axially extending components (69) of two adjacent aperture elements (67a, 67b, 67c, 67d) are configured staggerable.

2. The X-ray emitter (9a, 9b) according to claim 1, **characterized in that** at least two aperture pairs (67a, 67b, 67c, and 67d) are provided which are adjustable in different directions.

3. The X-ray emitter (9a, 9b) according to claim 1 or 2, **characterized in that** the beam forming aperture (57) is arranged at the collimator (50) as a collimator aperture.

4. The X-ray emitter (9a, 9b) according to one of the preceding claims, **characterized in that** the collimator (50) includes a collimation device (59) and the beam forming aperture (57) is arranged between the X-ray tube and the collimation device (59).

5. The X-ray emitter (9a, 9b) according to one of the preceding claims, **characterized in that** the beam forming aperture (57) is arranged rotatable relative to the X-ray tube, wherein the rotatability is provided in particular about the central beam axis (29, 31) of the X-ray tube.

6. The X-ray emitter (9a, 9b) according to claim 5, **characterized in that** the axially extending components (69) of the adjacent aperture elements (67a, 67b, 67c, 67d) include teeth (75a, 75b) which mesh with each other.

7. The collimator (50) for the X-ray emitter (9a, 9b) according to one of the preceding claims, wherein the collimator (50) includes at least one collimator aperture (57) which includes at least one aperture pair (67a, 67b, 67c, 67d) which is asymmetrically adjustable with reference to a central beam (29, 31) of the X-ray tube, **characterized in that** at least one aperture pair aperture pair (67a, 67b, 67c, 67d) extends in a lateral direction (71) and in an axial direction (69), **characterized in that** the axially extending components (69) of two adjacent aperture elements (67a, 67b, 67c, 67d) are configured staggerable.

8. The collimator (50) according to claim 7, **characterized in that** at least two aperture pairs (67a, 67b, 67c, 67d) are provided which are adjustable in different directions.

9. The collimator (50) according to one of the claims 7 or 8, **characterized in that** the collimator (50) includes a collimation device (59) and the collimator aperture (57) is arranged in front of the collimator device (59) with reference to the X-ray entry direction.

10. The collimator (50) according to one of the claims 7 - 9, **characterized in that** the collimator aperture (57) is arranged rotatable with reference to the X-ray tube, advantageously also with reference to the collimation device (59).

11. An X-ray work station (1), comprising at least one X-ray emitter (9a, 9b), an X-ray detector (11 a, 11 b, 19), a device for defining an arrangement position for an object (21, 37, 43) to be examined, for example a patient stretcher or similar, a suitable processing and control device and an output device (61), for example a printer, storage device or a screen, **characterized in that** the X-ray emitter (9a, 9b) is configured with features according to one of the claims 1- 6 and/or the collimator (50) is configured with features according to one of the claims 7 - 10.

12. A method for operating a X-ray emitter (9a, 9b), a collimator (50), an X-ray emitter (9a, 9b) or a x-ray work station (1), comprising at least one X-ray emitter (9a, 9b), an X-ray detector (11a, 11b, 19), a device for defining an arrangement position for an object (27, 37, 43) to be examined, a suitable processing and control device and an output device (61), wherein at least two aperture elements (67a, 67b, 67c, 67d) of a beam firming aperture (57) of the X-ray emitter (9a, 9b) are asymmetrically adjusted with respect to a central beam (29, 31) of the X-ray tube, **characterized in that** at least one aperture element (67a, 67b, 67c, 67d) extends in a lateral direction (71) and in an axial direction (79), wherein the axially extending components (69) of two adjacent aperture elements (67a, 67b, 67c, 67d) are staggered.

13. The method according to claim 12, **characterized in that** the X-ray emitter (9a, 9b) is configured with features according to one of the claims 1 - 8 and/or the collimator (50) is configured with features according one of the claims 9 - 12.

## Revendications

1. Source de rayons X (9a, 9b) comprenant un tube à rayons X, un boîtier, un écran, un diaphragme de mise en forme de faisceau (57) et un collimateur (50), le diaphragme de mise en forme de faisceau (57) comportant au moins une paire de diaphragmes (67a, 67b, 67c, 67d) qui est réglable de manière dissymétrique par rapport à un faisceau central (29, 31) du tube à rayons X, **caractérisé en ce qu'**au moins un élément de diaphragme (67a, 67b, 67c, 67d) s'étend dans une direction latérale (71) et une direction axiale (69), caractérisée que les parties (69), s'étendant axialement, de deux éléments de diaphragme (67a, 67b, 67C, 67d) disposés de manière adjacente sont conformés de manière à pouvoir s'entrecroiser.

2. Source de rayons X (9a, 9b) selon la revendication 1, **caractérisée en ce qu'**au moins deux paires de diaphragme (67a, 67b, 67C, 67d) sont prévues qui sont réglables dans des directions différentes.

3. Source de rayons X (9a, 9b) selon la revendication 1 ou 2, **caractérisée en ce que** le diaphragme de mise en forme de faisceau (57) est disposé au niveau du collimateur (50) en tant que diaphragme de collimateur.

4. Source de rayons X (9a, 9b) selon l'une des revendications précédentes, **caractérisée en ce que** le collimateur (50) comprend un moyen de collimation (59) et le diaphragme de mise en forme de faisceau (57) est disposé entre le tube à rayons X et le moyen de collimation (59).

5. Source de rayons X (9a, 9b) selon l'une des revendications précédentes, **caractérisée en ce que** le diaphragme de mise en forme de faisceau (57) est disposé de manière à pouvoir tourner par rapport au tube à rayons X, la capacité de rotation s'effectue particulier sur l'axe de faisceau central (29, 31) du tube à rayons X.

6. Source de rayons X (9a, 9b) selon la revendication 5, **caractérisée en ce que** les parties (69), s'étendant axialement, d'éléments de diaphragme (67a, 67b, 67C, 67d) disposés de manière adjacente comportent des dents (75a, 75b) qui engrènent entre elles.

7. Collimateur (50) destiné à la source de rayons X (9a, 9b) selon l'une des revendications précédentes, le collimateur (50) comporte au moins une diaphragme de collimateur (57) qui comporte au moins une paire de diaphragmes (67a, 67b, 67c, 67d) qui est réglable de manière asymétrique par rapport à un rayon central (29, 31) du tube à rayons X, **caractérisé en ce qu'**au moins un élément de diaphragme (67a, 67b, 67c, 67d), s'étend dans une direction latérale (71) et une direction axiale (69), **caractérisé en ce que** les parties (69), s'étendant axialement, de deux éléments de diaphragment (67a, 67b, 67C, 67d) disposés de manière adjacente sont conformées de manière à s'entrecroiser.

8. Collimateur (50) selon la revendication 7, **caractérisée en ce qu'**au moins deux paires de diaphragmes (67a, 67b, 67C, 67d) sont prévues qui sont réglables dans des directions différentes.

9. collimateur (50) selon l'une des revendications 7 ou 8, **caractérisé en ce que** le collimateur (50) comprend un moyen de collimation (59) et le collimateur de diaphragme (57) est disposé en amont du moyen de collimation (59) par rapport à la direction d'entrée des rayons X.

10. Collimateur (50) selon l'une des revendications 7 à 9, **caractérisé en ce que** le collimateur de diaphragme (57) est disposé de manière rotative par rapport au tube à rayons X, également de préférence au niveau du moyen de collimation (59).

11. Poste de travail radiographique (1), comprenant au moins une source de rayons X (9a, 9b), un détecteur de rayons X (11a, 11b, 19), un dispositif destiné à définir une position d'agencement d'un objet à examiner (21, 37, 43), par exemple une surface de support de patient ou analogue., un dispositif de traitement et de commande approprié et un dispositif de sortie (61) tel qu'une imprimante, un moyen de mémorisation ou un écran, **caractérisé en ce que** la source à rayons X (9a, 9b) présente les caractéristiques selon l'une des revendications 1 à 6 et/ou le collimateur (50) présente les caractéristiques selon l'une des revendications 7 à 10.

12. Procédé pour faire fonctionner une source à rayons X (9a, 9b), un collimateur (50) d'une source de rayons X (9a, 9b) ou un porte de travail radiographique (1), l'au moins source de rayons X (9a, 9b), un détecteur de rayons X (11a, 11b, 19), un dispositif destiné à définir une position d'agent d'un objet à examiner (21, 37, 43), un dispositif de traitement et de commande approprié et un dispositif de sortie (61), au moins deux éléments de diaphragme (67a, 67b, 67C, 67d) d'un diaphragme de mise en forme de faisceau (57) de la source de rayons X (9a, 9b) étant réglés de manière asymétrique par rapport à un faisceau central (29, 31) d'un tube à rayons X, **caractérisé en ce qu'**au moins un élément de diaphragme (67a, 67b, 67c, 67d) s'étend dans la direction latérale ((69) et 71) et la direction axiale (69), les parties (69), s'étendant axialement, de deux éléments de diaphragme adjacents (67a, 67b, 67c, 67d) s'entrecroisant.

13. Procédé selon la revendication 12, **caractérisé en ce que** la source de rayons X (9a, 9b) présente les caractéristiques selon l'une des revendications 1 à 8 et/ou le collimateur (50) présente les caractéristiques selon l'une des revendications 9 à 12.
